# EUROPEAN PATENT APPLICATION

(11) **EP 0 625 357 A1**
(43) Date of publication of application: **23.11.1994**
(21) Application number: 93500170.1
(22) Date of filing: 22.12.1993
(51) Int. Cl.: A61M 1/00

(54) **A pleural drainage unit**

(30) Priority: 20.05.1993 ES 9301090
(71) Applicant: Estrada Salo, Gaspar, E-08450 Llinars del Valles (Barcelona) (ES); Gomez Sebastian, Guillermo, E-08029 Barcelona (ES); Leon Gonzalez, Carlos, E-08009 Barcelona (ES)
(72) Inventor: Estrada Salo, Gaspar, E-08450 Llinars del Valles (Barcelona) (ES); Gomez Sebastian, Guillermo, E-08029 Barcelona (ES); Leon Gonzalez, Carlos, E-08009 Barcelona (ES)
(74) Representative: Gomez-Acebo y Pombo, José Miguel

(57) **Abstract**

A pleural drainage unit comprising three chambers (1, 2 and 3) of upwardly decreasing cross-section and with opposable lateral faces which join together to form an assembly of approximately semi-trunco-conical configuration which includes a reversible handle (14). The first chamber (1), for collecting liquids,, is provided on its upper with a flexible nozzle (12) to which the patient's probe is connected, and on its front wall a level indicator (13). The second and third chambers (2 and 3) are provided with means of checking the level of liquid inside as well as caps for closure and connection to a vacuum system.

## Description

The present invention relates to a pleural drainage unit designed to collect the liquids and gases which may be produced in the pleural cavity in certain patients or in certain situations.

The pleural drainage units consist three chambers, a first chamber for collecting pleural liquids, a second chamber for sealing and a third chamber for suction control. The first and second chambers are interconnected by means of a tube which leads from the first chamber, at a point above the maximum level which the liquids can reach in said chamber, and opens in the second chamber, at a point close to the bottom thereof. There is an interconnection between the second and third chambers between points situated above the maximum level of liquid in said chambers. Furthermore, the third chamber is provided with a tube which extends nearly to the bottom of said chamber and which opens above to the exterior.

### BACKGROUND OF THE INVENTION

The units of this type currently in use constitute excessively bulky assemblies which are difficult for the patient to transport and which furthermore are not provided with easily readable check elements.

However, it is desirable that these pleural drainage units allow both the checking of their operation as well as the reading of the levels to be carried out simply and rapidly, and furthermore that they can be transported comfortably by the patient who has to live with the unit for a certain period of time. In other words, the drainage unit should satisfy a number of ergonomic factors as far as both the patient and the second operator in charge of checking the unit are concerned.

The object of the present invention is to develop an autonomous pleural drainage unit which is constituted in such a way that the various component parts form a stable assembly which can be transported easily by the patient and which is provided with means for making it easy to handle, check and position the unit, at the same time as ensuring perfect operation in the purpose for which said unit is designed.

### DESCRIPTION OF THE INVENTION

According to the present invention the three chambers which form the drainage unit are of upwardly decreasing cross-section and have opposable lateral faces which join together to form an approximately semi-trunco-conical assembly provided with a reversible handle or hook.

The integration of the various parts or components of a single assembly constitutes one of the essential design characteristics of the unit of the invention since it enables the separation of the connections to be reduced, thereby reducing to a minimum any lack of watertightness of the system.

Furthermore, as a direct result of integrating the various parts in a single body, a highly stable structure is achieved. This characteristic is further helped by the fact that the centre of gravity of the assembly is very low, the lower part being substantially wider than the upper part, almost all of the liquid thereby being contained in the lower part when the unit is in use and giving it maximum stability.

The first chamber of the unit is provided on its upper face with a flexible nozzle to which is connected the pleural probe. The flexibility and orientation ability of this nozzle prevents the pleural drain from kinking where it joins to the liquid collection chamber, making it unnecessary to add an anti-kinking mechanism to the pleural drain at this point.

The first chamber of the drainage unit is further provided with a level indicator situated on the front wall. This level indicator consists of one or more channels which run vertically over the front surface of the first chamber and which are transparent in nature, enabling the level of liquid inside said chamber to be seen. Furthermore, an indicator or marker can be fitted inside the channels, moved manually along said channel and elastically fixed to the surface thereof in order to mark the previous reading taken, the time and date of said reading being marked on the external surface of the part which forms the indicator. A clip joined to the pleural probe enables it to be fixed to the clothes, preventing it from falling onto the floor and forming the ideal slope to ensure a correct drainage operation.

The second and third chambers are provided with means of checking the level of liquid inside, a cap for closing the chamber, a connection to a vacuum system and an inter-chamber pressure increase safety valve.

The second chamber may be subdivided into two compartments, a lower one of large dimensions which is the one connected to the first and third chambers, and an upper one of lower capacity which connects to the lower compartment by means of a valve which can be operated from the outside. The size of the upper compartment is such that it can contain the quantity of liquid which the lower compartment will need when the drainage unit is in use.

Like the second chamber, the third chamber of the unit may be also be subdivided into two compartments which fit the same description.

It is very important that no noises are produced in the suction chamber due to air circulation. This can be achieved by making the lower part of the tube, which connects with the outside, thinner such that any air bubbles which might circulate are small and of regular shape.

The subdivision of the second and third chambers into two compartments makes it possible to provide each chamber with a self-refill system. In this way the time taken to prepare and maintain the drain is very short. The liquid contained in the upper compartment can be totally or partially emptied into the lower compartment, depending on the requirements at the time the unit is put into operation. Generally, the upper compartment of the second chamber is emptied totally into the lower compartment since the amount of liquid needed in this second chamber is constant. The level of liquid in the third chamber, however, may vary depending on the emptying requirements of the patient.

The incorporation of the self-refill system described makes the new system autonomous, since the drainage unit is supplied with the liquid required to put it into operation at any time without the help of an external supply of liquid, at the same time simplifying and perfecting the handling of the unit by the medical staff. Furthermore, the possibility of error is reduced since it is easier to put the unit into operation with the necessary levels of liquid in the chambers.

### BRIEF DESCRIPTION OF THE DRAWINGS

In order that the characteristics of the present invention be better understood, the accompanying drawings show by way of a non-limiting example one practical embodiment thereof. In the drawings:
Figure 1 is a constitutional and functional diagram of a traditional pleural drainage unit.
Figure 2 in a front elevation of a pleural drainage unit according to the invention.
Figure 3 is an upper plan view of the unit shown in figure 2.
Figure 4 is a similar elevation to that of figure 2 showing an alternative embodiment.
Figure 5 is an upper plan view of the unit shown in figure 4.
Figure 6 is a right lateral elevation of the unit shown in figure 4.
Figure 7 is a left lateral elevation of the unit shown in figure 4.
Figure 8 is a schematic vertical section of the second and third chambers.
Figure 9 is a section similar to that of figure 8 showing the upper parts of the second and third chambers.

### DESCRIPTION OF A PREFERRED EMBODIMENT

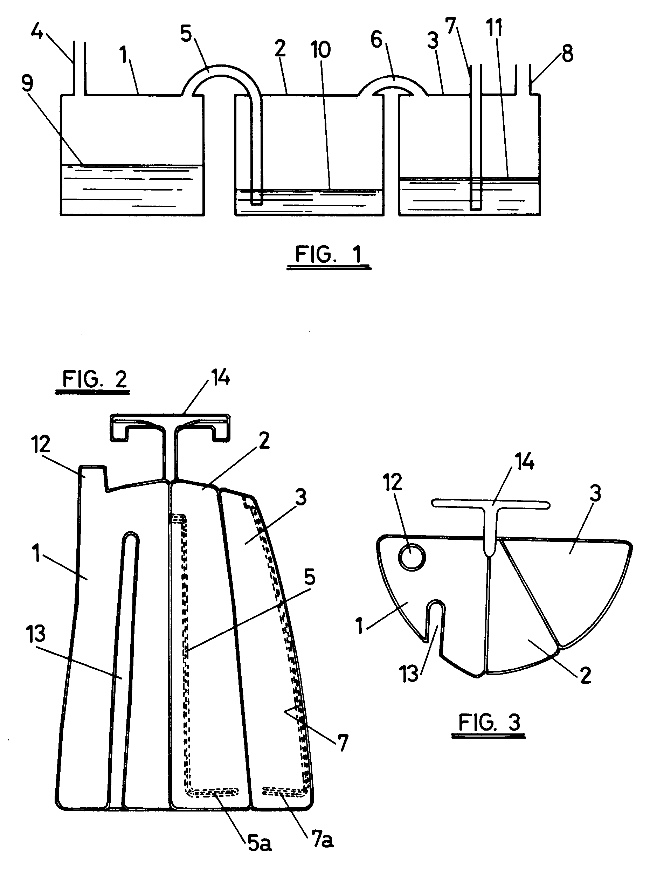

Figure 1 shows a schematic representation of a traditional pleural drainage unit comprising three chambers, a first chamber 1 for collecting pleural liquids, a second chamber 2 for sealing and a third chamber 3 for suction. A probe 4 coming from the patient is connected to the first chamber 1. The chambers 1 and 2 are inter-connected by means of a tube 5 which leads from the upper part of tank 1 to a point close to the bottom of tank 2. Chambers 2 and 3 are interconnected above by means of a tube 6. The suction chamber 3 is further provided with a tube 7 which leads from a point close to the bottom of said chamber and opens above to the outside. Finally, the suction chamber is provided with a nozzle 8 to which is connected the vacuum system of the hospital or medical centre.

The liquids or gases which may be sucked in arrive at the chamber 1 reaching a level 9. The sealing chamber 2 contains water or similar up to a constant level 10, below which the tube 5 opens. Finally, the suction control chamber 3 contains a variable level of water, serum or similar, depending on the degree of suction via the tube 8, the condition of the patient, etc.

According to the invention, the assembly of chambers and components described comprises a unit which is shown in figures 2 and 3, in which the same reference numbers as figure 1 are used for the same components.

In the embodiment shown in the drawings, the chambers 1, 2 and 3 are of upwardly decreasing cross-section and have opposable lateral faces which can be joined together, by means of adhesive substances for example, to form a unit whose general configuration is approximately semi-trunco-conical.

Chamber 1 is provided on its upper face with a flexible nozzle 12, to which is connected the pleural probe which is easy to orientate due to the flexibility of said nozzle 12. The front wall of chamber 1 is provided with at least one transparent, vertical channel 13 which enables the level of liquid inside said chamber to be seen.

The interconnection between chambers 1, 2 and 3 is carried out via the common walls of said chambers which are in contact. The assembly is provided with a reversible hook 14 which enables it to be hung anywhere, when the patient is lying down, or which can be used as a carrying handle to transport the assembly.

As can be seen in figure 2 the interconnection tube 5 between chambers 1 and 2 terminates at its lower end in a kinked section 5a which runs close to the bottom of chamber 2, approximately parallel thereto. In the same way, the tube 7 of chamber 3 terminates at its lower end in a section 7a which runs parallel to and close to the bottom of said chamber.

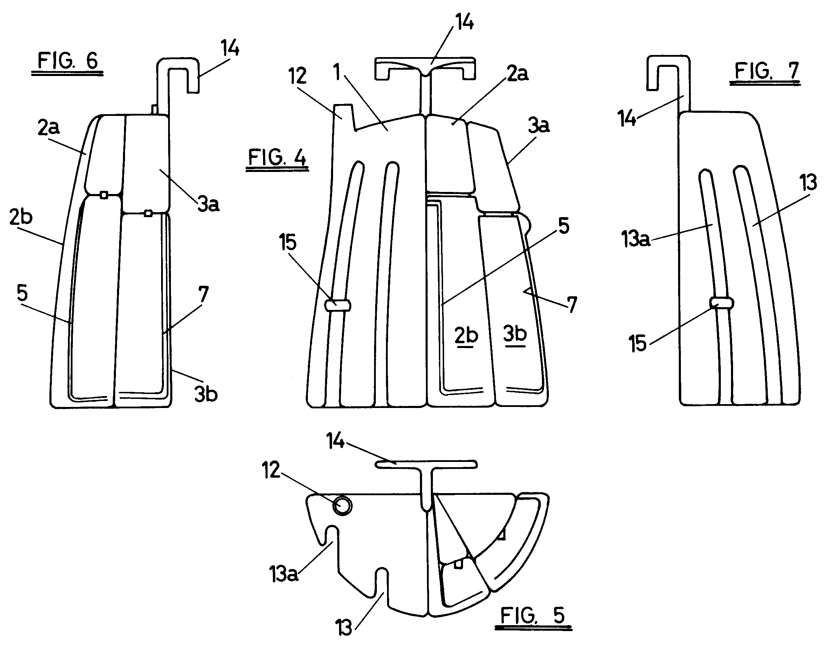

Preferably, the second chamber 2 for sealing is subdivided into two compartments, an upper one 2a and a lower one 2b, figure 4, the second of these compartments being the one which contains the liquid below the level of which opens the tube 5.

In the same way, the third chamber 3 for suction control is subdivided into two compartments, an upper one 3a and a lower, larger one 3b which contains the liquid from below the level of which leads the tube 7 which opens to the outside.

The first chamber 1 for liquid collection may be provided on its frontal wall with two channels, 13 and 13a, with transparent bases, for reading the level of drained liquid. One of these channels, for example channel 13a, can be provided with an indicator 15 consisting of a plate provided on its rear surface with elastic feet which can be introduced, and thereby fixed, into the channel 13a. The indicator 15 is fixed at the level reached by the liquid inside the chamber 1 the last time a reading was made. The time and date of said last reading can be written on the external plate of the indicator.

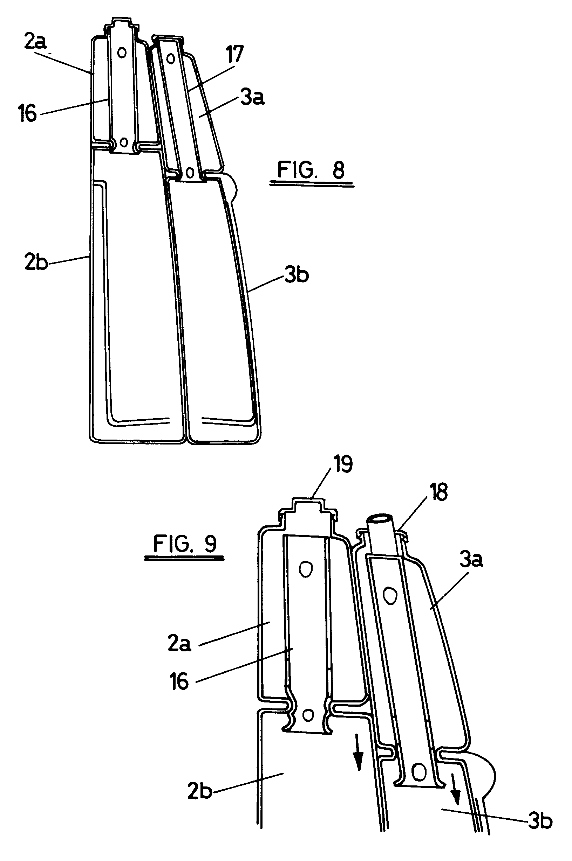

The compartments 2a and 3a are designed to contain the quantity of which has to be emptied into the lower compartments 2b and 3b during the operation of the assembly. As can be seen in figures 8 and 9, the upper camber 2a and the lower chamber 2b are joined via a valve 16 which can be operated from the upper part of the chamber 2b. In the same way the chambers 3a and 3b are joined via a valve 17 which can also be operated from the upper part of the chamber 3a.

Before the unit is connected the valves 16 and 17 are moved until they the position shown in figure 9 where the liquids contained in the upper chambers 2a and 3a pass into the lower chambers 3a and 3b. The amount of liquid which passes from 3a to 3b can be regulated, to adjust the level reached by the liquid in chamber 3, depending on the degree of suction or the condition of the patient.

The vacuum system of the hospital or medical centre is connected to the chamber 2a by means of the connection 19 thereof. The chamber 3a has a hermetic sealing cap 18 which is essential for its suction control operation.

The valve 16 must remain open whilst the system is in operation so once it has been used to fill the chamber 2b with liquid, it cannot return to the closed position between the chambers 2a and 2b. The valve 17 can be closed such that the chamber 3a can be filled as desired.

With this arrangement it is possible to carry out a self-refill of the chambers 2 and 3, making the unit autonomous as far the supply of the necessary serum and liquids is concerned, at the same time simplifying and improving the handling of the unit.

The horizontal sections 5a and 7a of the tubes 5 and 7 increase the time it takes for the air bubbles to pass, thereby making it easier to observe the air losses of the patient. Furthermore, the section 7a, which has a narrow cross-section, reduces any noise caused by the entrance of air.

The arrangement described results in integrated assembly which is easy to handle, can be easily transported by the patient and which is highly stable as a result of its general configuration.

## Claims

1. A pleural drainage unit comprising three independent chambers, a first chamber for collecting pleural liquids, a second chamber for sealing and a third chamber for suction control, said chambers being consecutively interconnected by means of a first interconnection which leads from the first chamber, from a point situated above the maximum level which the liquids can reach, and opens in the second chamber, at a point close to the bottom, and by means of a second interconnection which runs between points of the second and third chambers situated above the maximum level of liquid in said chambers, the third chamber being further provided with a tube which leads from a point close to the bottom and opens above to the outside, characterized in that the three chambers are of upwardly decreasing cross-section and have opposable lateral faces which join together to form an assembly of approximately semi-trunco-conical configuration, provided with a reversible handle or hook and a clip for fixing the probe to the clothes, the first chamber being provided on its upper face with a flexible nozzle to which the patient's probe is connected, and on its front wall a level indicator, the second and third chambers being provided with means of checking the level of liquid inside as well as caps for closure and connection to a vacuum system.

2. A unit according to claim 1, characterized in that the first chamber for collecting liquids is provided on its front wall with at least one transparent, vertical channel in which an indicator is externally mounted and can moved and fixed elastically along said channel.

3. A unit according to claim 1, characterized in that the second and third chamber are subdivided into two compartments, a lower one of large dimensions designed to contain the liquid necessary for the operation of the assembly, and an upper one of lower capacity which is connected to the lower compartment by means a valve which can be operated from the outside, the size of the upper compartment being such that it can contain the quantity of liquid which the lower compartment will need when the assembly is in use.

4. A unit according to claim 1, characterized in that the interconnection tube between the first and second chambers, and the tube which leads from the third chamber to the outside, are each provided with a long, lower section which runs parallel to and close to the bottom of the corresponding chamber.
